# EUROPEAN PATENT APPLICATION

(11) **EP 2 711 027 A1**
(43) Date of publication of application: **26.03.2014**
(21) Application number: 13168739.4
(22) Date of filing: 22.05.2013
(51) Int. Cl.: A61L 2/10, A61L 2/26

(54) **Ultraviolet sterilizer for portable digital devices and accessory parts**

(30) Priority: 21.09.2012 RU 2012140355; 21.09.2012 RU 2012140356 U
(71) Applicant: Abramov, Timofey Adamovich, Moscow 125414 (RU)
(72) Inventor: Abramov, Timofey Adamovich, Moscow 125414 (RU)
(74) Representative: Beetz & Partner

(57) **Abstract**

The invention relates to devices for the protection against bacteria and microorganisms by means of sterilization of portable digital devices and accessory parts. The sterilizer comprises a case, a protective cover (9) and sources of ultraviolet radiation (5). The sources of ultraviolet radiation (5) are fixed on two opposite walls (3) inside the case. The case walls (3) are provided with reflectors (4). The sterilizer further comprises a container (7) for receiving the portable digital devices and accessory parts to be disinfected. The container (7) is designed to be permeable for ultraviolet radiation and makes it possible to actuate the device, so that the sources of ultraviolet radiation (5) can be switched on.

## Description

The invention relates to devices for the protection against bacteria and microorganisms by means of sterilization of portable digital devices and the pertaining accessories.

There has already been known a number of devices for the disinfection of digital devices including mobile phones.

In printed publication KR20030033475 (A) of 01.05.2003 there has been disclosed a portable mobile phone charging device for automatically sterilizing mobile phones when the phone has been placed in the charging device. This portable charging device represents a device into which the mobile phone battery is introduced for being charged. One or more charging state indicator lamps (112) are working, while the mobile phone is being charged. An ultraviolet lamp is connected in parallel with the charging state indicator lamp so that the ultraviolet lamp is automatically switched on when the charging state indicator lamp is switched on.

The present device is only intended for the sterilization of mobile phones and not for any other electronic devices and requires connection to the electric network.

From printed publication CN2762808 (Y) of 08.03.2006 there has been known a microcomputer-controlled aromatizing, cleaning and sterilizing device for a cellular telephone. This is a tool which is specifically used for cleaning and sterilizing a cellular telephone. The device case is divided into two parts and is equipped with an ultraviolet lamp, a miniature air heater and an automatic control unit.

This device has a complex structure and is not adapted for the sterilization of small accessory parts such as ear-phones.

The utility model CN2922904 (Y) of 18.07.2007 discloses a sterilizer containing an ultraviolet disinfection device, an ozone generator and a case, with the ultraviolet disinfection device being placed in the upper part of the case and the ozone generator being provided in the bottom part of the case. The ultraviolet disinfection device and the device of ozone supply are respectively connected with the power supply. By means of the device it is conveniently possible to sterilize small objects including keys, cellular phones, portable computers, etc.

The sterilizer does not contain any convenient holder or container for accommodating the objects to be treated. Moreover, the placement of the ultraviolet disinfecting device in the upper part of the case will prevent the object from being treated with ultraviolet radiation from different directions.

The prior art which comes closest to the invention is the sterilizer for mobile phones disclosed in printed publication CN 201026311 Y published on 27.02.2008.

The known sterilizer has a case, and the ultraviolet lamps are fixed on two opposite walls inside the case and provided with reflectors. In the upper part of the case there is placed a protective cover with a handle.

The shortcomings of this closest prior art consist in the inconvenience of accommodating the portable digital devices and accessory parts to be sterilized inside the case; moreover, there is no operation indicator for the ultraviolet lamps.

The object of this invention resides in providing a convenient universal sterilizer for the disinfection of portable digital devices and accessory parts.

The technical problem underlying the invention resides in that the disinfection can be effected more reliably owing to the exact positioning of the digital devices and accessory parts relative to the sources of the ultraviolet radiation.

The technical problem is solved by the present device owing to the fact that the sterilizer comprising a case, a protective cover, and sources of ultraviolet radiation which are fixed on the two opposite walls inside the case and provided with reflectors, further comprises a container for receiving the portable digital devices and accessory parts to be disinfected, which is designed to be permeable for ultraviolet radiation, with the container being adapted for actuating the device, so that the sources of ultraviolet radiation can be switched on.

The technical problem is further solved by the fact that the sources of ultraviolet radiation are placed in the upper part of the case, and the bottom part of the case comprises a seat for the batteries and a switch for switching on the sources of ultraviolet radiation; the protective cover is UV-opaque and provided with a display area for the switch-on state of the sources of ultraviolet radiation. Besides that, the sources of ultraviolet radiation can be designed as an ultraviolet ozone tube or as a quartz lamp; furthermore the container for receiving the portable digital devices and accessory parts to be disinfected shall have a weight being sufficient for actuating the device, so that the sources of ultraviolet radiation can be switched on.

The present invention is explained on the basis of the following schematic Figures:

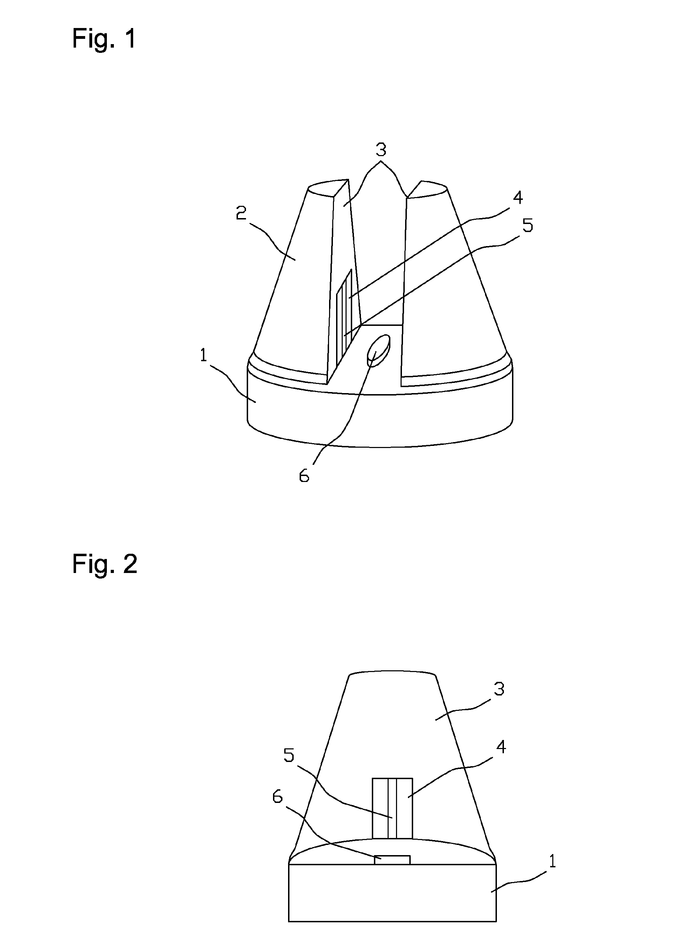

Fig. 1 shows the sterilizer case without cover;
Fig. 2 shows the case in longitudinal section;

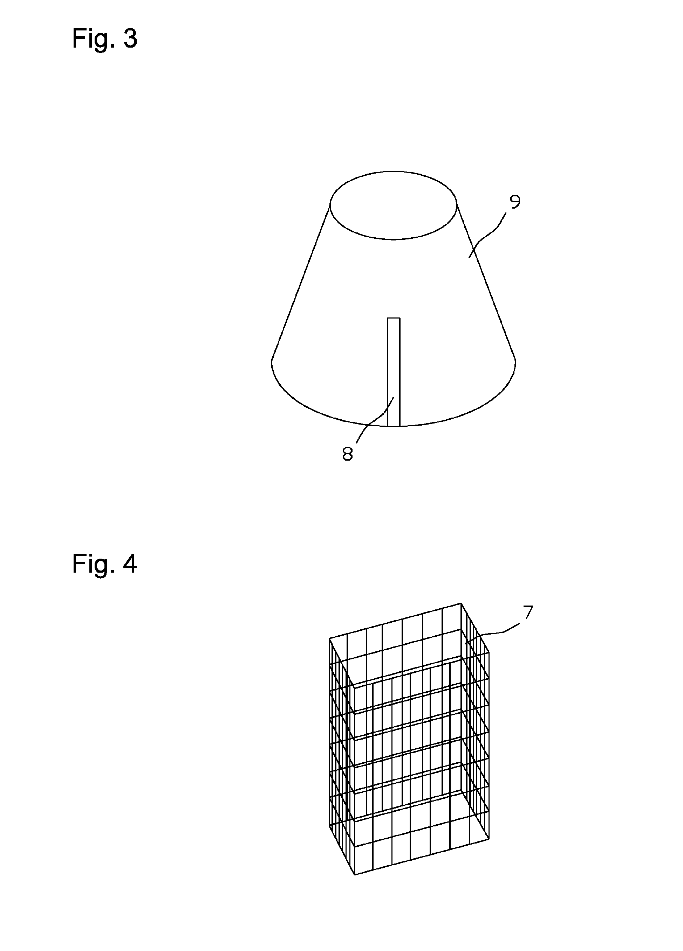

Fig. 3 shows the cover;
Fig. 4 shows the container for receiving the portable digital devices and accessory parts to be disinfected.

The sterilizer for portable digital devices and accessory parts comprises a case consisting of a bottom part 1 and an upper part 2. At the opposite inner walls 3 of the upper part 2 of the case there are placed reflectors 4 and ultraviolet lamps 5. In the bottom part 1 of the case there are disposed the electric circuit connected with the lamps 5 for the ultraviolet radiation as well as the seat for the batteries or accumulators (not shown in the Figure). A switch 6 for switching on the ultraviolet lamps 5 is positioned on the bottom part 1 of the case in the recess for receiving the container 7 for the objects to be disinfected. The cover is equipped with a display area 8 for the switch-on state of the ultraviolet lamps 5.

The portable digital device such as, e.g., a mobile phone or a player is placed in the container 7. The container 7 is placed in the sterilizer case, whereby its weight actuates the switch 6 for switching on the ultraviolet lamps 5, so that the latter can be switched on thereby. Then, the cover is placed thereon. While the lamps 5 are operating, the indicator is glowing, making it possible to see, at closed cover, whether the process of disinfecting is in progress and to notice a low battery state early enough.

The container 7 makes it possible to position the mobile phone or the player to be disinfected relative to the ultraviolet lamps 5, so that the process can reliably be performed.

After the sterilization, the container 7 is removed from the case, whereby the switch 6 returns into its initial position and the ultraviolet lamps 5 are switched off.

## Claims

1. A sterilizer for portable digital devices and accessory parts, comprising a case, a protective cover (9), sources of ultraviolet radiation (5) fixed on two opposite walls (3) inside the case and provided with reflectors (4), **characterized in that** it further comprises a container (7) for receiving the portable digital devices and accessory parts to be disinfected, which is designed to be permeable for ultraviolet radiation, with the container (7) being adapted for actuating the device, so that the sources of ultraviolet radiation can be switched on.

2. The sterilizer according to claim 1, **characterized in that** the sources of ultraviolet radiation (5) are placed in the upper part (2) of the case.

3. The sterilizer according to claim 1 or 2, **characterized in that** the bottom part (1) of the case is equipped with a seat for the batteries and with a switch (6) for switching on the sources of ultraviolet radiation (5).

4. The sterilizer according to one of the preceding claims, **characterized in that** the cover (9) is designed to be UV-opaque and is provided with a display area (8) for the switching-on state of the sources of ultraviolet radiation (5).

5. The sterilizer according to one of the preceding claims, **characterized in that** the sources of ultraviolet radiation (5) are designed as an ultraviolet ozone tube or a quartz lamp.

6. The sterilizer according to one of the preceding claims, **characterized in that** the container (7) for receiving portable digital devices and accessory parts to be disinfected has a weight sufficient for actuating the device, so that the sources of ultraviolet radiation (5) can be switched on.
